# EUROPEAN PATENT SPECIFICATION

(11) **EP 2 710 609 B1**
(45) Date of publication and mention of the grant of the patent: **19.07.2017**
(21) Application number: 11860754.8
(22) Date of filing: 16.12.2011
(51) Int. Cl.: G21K 1/02, A61B 6/06

(54) **HOLDER FOR CYLINDRICAL BENDING OF AN X-RAY GRID**
HALTER FÜR ZYLINDRISCHES BIEGEN EINES RÖNTGENRASTERS
SUPPORT POUR LA COURBURE CYLINDRIQUE D'UNE GRILLE POUR RAYONS X

(30) Priority: 17.03.2011 SE 1100196
(43) Date of publication of application: 26.03.2014
(73) Proprietor: FlexGrid AB, 163 53 Spånga (SE)
(72) Inventor: NARFSTRÖM, Jan, 192 51 Sollentuna (SE); LANDSTRÖM, Stig, 184 51 Österskär (SE)
(86) International application number: PCT/SE2011/000231
(87) International publication number: WO 2012/125086

(56) References cited:
- DE-C1- 4 305 475
- JP-A- 2001 188 098
- JP-A- 2004 313 546
- JP-A- 2008 249 772
- US-A- 2 801 671
- US-A- 5 291 539
- US-A1- 2005 152 500
- US-A1- 2005 175 154
- US-A1- 2007 183 583

## Description

The present invention relates to a holder for an X-ray grid according to the independent claim. It relates to such a holder for an X-ray grid intended for cylindrically bending an X-ray grid.

### Background of the invention

US5291539 discloses a holder for an X-ray grid intended for cylindrically bending an X-ray grid. The holder holds the grid along two opposing sided and is provided with a mechanical arrangement for simultaneously twisting both sides upwards or downwards. The grid comprises rows of parallel X-ray absorptive layers, separated by layers which are more transparent to X-rays. The rows of layers therefore absorb X-rays that are not directed straight against the grid. By bending it, it does instead transmit X-rays emitted by a line source and by controlling the curvature of the grid, the distance to the line source whose X-rays are transmitted may be selected.

The grid may comprise lead layer and it has a comparatively large weight. The grid therefore tends to deviate from an optimal cylindrical shape and it will then partially absorb radiation that should have been transmitted and it will also transmit radiation that should have been absorbed. As the grid is held only along two opposite sides, it tends to be sensitive to vibrations.

Another holder for an X-ray grid is disclosed by SE520527C2 where the grid is held between three rows of inflatable air cushions arranged in parallel to each other. The air cushions along the middle line is inflated such that the mid portion of the grid is lowered or raised, while the air cushions along the other lines forces the grid in the opposite direction. The air cushions may give a deviation from the desired, cylindrical, shape and the grid may remain fully plane along its outer portions.

US2801671 discloses a method of producing a focused X-ray grid, according to which a conveyor is provided to support and form a sandwich or compressed structure having the arcuate shape of the conveyor. This X-ray grid is a rigid element having bent shape with a fixed radius selected at manufacturing, giving it a fixed and unchangeable focal length.

An object of the invention is therefore to provide a holder for an X-ray grid which better approximates part of a cylinder.

Another object of the invention is therefore to provide a holder for an X-ray grid that is able to hold an X-ray grid bent in a fixed shape, no matter how the holder for an X-ray grid is mounted and unaffected by vibrations.

These and other objects are attained by a holder for an X-ray grid according to the independent claim.

### Summary of the invention

The invention relates to a holder for cylindrical bending of an X-ray grid 15, where the X-ray grid holder supports the X-ray grid with supporting elements 5a-b, 7a-b, 8a-b, 9a-b, 10a-b arranged along five supporting lines 14a-e being parallel to and separated from each other. The supporting elements are constituted by moveable supporting elements 8a-b, 9a-b, 10a-b and fixed supporting elements 5a-b, 7a-b. Moveable supporting elements 8a-b positioned along amid supporting line 14c are arranged to raise when moveable supporting elements 9a-b, 10a-b positioned along outermost supporting lines 14a-b are lowered, while fixed supporting elements 5a-b, 7a-b positioned along the remaining two supporting lines stay fixed. With supporting elements distributed over five support lines, the grid may be made to precisely follow a desired cylindrical curvature.

In an advantageous embodiment, the moveable supporting elements 9a-b, 10a-b are moveably arranged along the sides of the grid holder and are provided with devices 23 for achieving raising or lowering of the grid when the moveable supporting elements are moved along the sides of the grid holder. The devices 23 for achieving raising or lowering of the grid may be constituted by slanted grooves 23 in the moveable supporting elements, where the grooves are arranged to cooperate with pegs 16a-b, 17a-b, 18a-b on the grid.

In another advantageous embodiment, the moveable supporting elements 9a-b, 10a-b are mechanically connected to each other, such that all the moveable supporting elements move simultaneously. The fixed supporting elements 5a-b, 7a-b may be constituted by rails at the bottom and at the lid of the grid holder facing each other.

### Brief description of the drawings

Fig. 1 shows the bottom and the lid of the X-ray grid holder without the grid
Fig. 2 shows the bottom and the lid of the X-ray grid holder with the grid
Fig. 3 shows the X-ray grid
Fig. 4 shows a linear actuator
Fig. 5 shows part of the frame of the X-ray grid holder with a corner wheel

### Description of a preferred embodiment

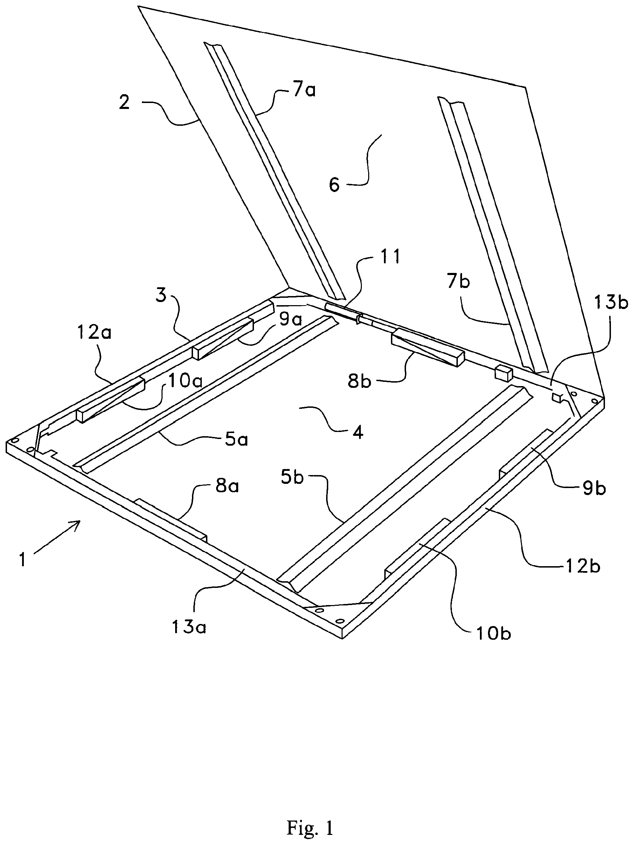

Fig. 1 shows the bottom 1 and the lid 2 of the X-ray grid holder without the grid. The bottom comprises a rectangular frame 3 arranged on a rectangular bottom plate 4. Above the bottom plate two bottom rails 5a-b extends in parallel to each other. The bottom rails are arranged a distance from respective opposite sides of the frame corresponding to approximately one quarter of the width of the bottom plate. The lid comprises a lid plate 6 across which two lid rails 7a-b extend in parallel to each other. The lid plate is attached onto the frame such that the lid rails are positioned straight above the bottom rails, and the lid rails are in a corresponding fashion arranged a distance from respective opposite sides of the lid plate corresponding to approximately one quarter of the width of the lid plate. With the lid mounted onto the bottom there is a space between the rails on the lid and the bottom set such that the grid fits between the rails.

Along the interior side of the frame six linear actuators 8a-b, 9a-b, 10a-b are arranged, two 8a-b near the midpoint of a first pair of opposite sides 12a-b and four 9a-b, 10a-b on the second pair of opposite sides 13a-b. The four linear actuators 9a-b, 10a-b on the second pair of opposite sides are arranged a distance approximately corresponding to one third of the length of the second side from respective ends of the second side. All the linear actuators are arranged to be moveable in grooves along the sides at which they sit. The movement is achieved with a control unit 11 that linearly moves the linear actuators and all the linear actuators are attached to each other via a cable such that they move synchronously with each other.

Diagonally across the front sides of the six linear actuators, ie. the sides facing inwards, extend grooves that receive six corresponding pegs on the X-ray grid. When the linear actuators are displaced along the sides of the frame, the pegs on the X-ray grid is correspondingly displaced upwards or downwards, such that the grid is bent in a curve parallel to one side of the grid.

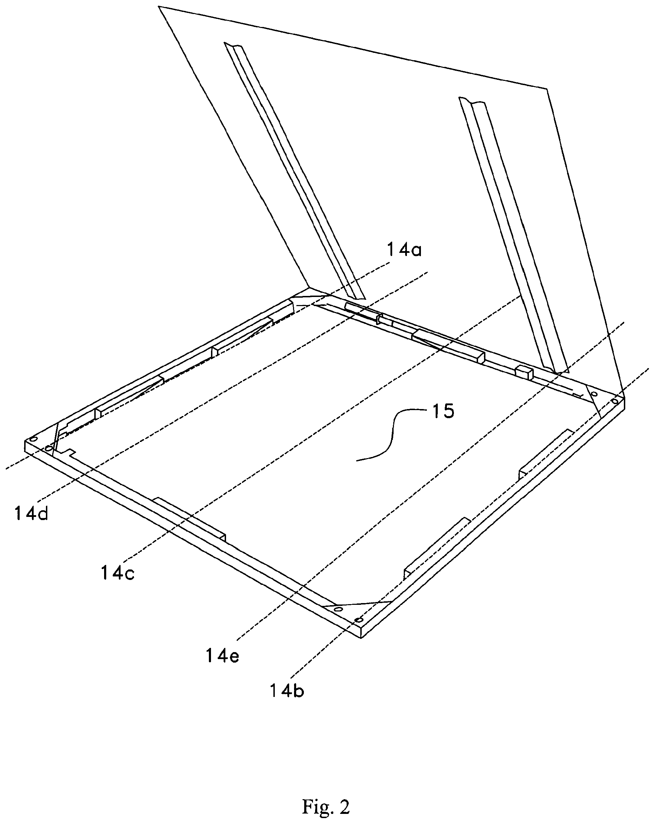

Fig. 2 shows the bottom and the lid of the X-ray grid holder with the X-ray grid 15 mounted in the bottom. The grid is thus in the figure held at six points along its periphery, where the pegs of the grid engages the grooves of the linear actuators. The six holding points are distributed over three parallel, moveable supporting lines 14a-c, where two outer moveable supporting lines 14a-b sits immediately by and extends in parallel to the first pair 12a-b of opposite sides of the frame, and the third, middle, moveable line 14c extends between the midpoints of the second pair of opposite sides 13a-b.

In addition to the three moveable, parallel lines, the grid is held in place along two further, fixed, supporting lines 14d-e that are constituted by the bottom and lid rails 5a-b, 7a-b. The bottom and lid rails to extend in parallel with the moveable supporting lines and the rails are arranged on each side of the middle moveable supporting line and inside the outermost moveble supporting lines.

As the control unit displaces all the linear actuators, the moveable supporting lines are linearly displaced up and down, respectively, depending on in which direction the displacement takes place, and depending on in which way the diagonal grooves in the linear actuators are arranged. To bend the grid into a convex shape, all the linear actuators are displaced counter clockwise. During such a counter clockwise movement, both the outer supporting lines are lowered towards the bottom plate 4, the middle moveable supporting line is raised from the bottom plate and both the fixed supporting lines remains in place. The grid is then forced to adopt a convex cylindrical shape that is defined by all the five supporting lines 14a-e. In order to not force the grid to form an inflexion point at both the fixed supporting lines 14d-e, which would had been the case if the rails had flat upper surfaces, the rails are in cross section rounded with a steeply sloping tip.

In order to bend the grid into a concave shape, all the linear actuators are displaced clockwise.

During such a clockwise movement, both the outer moveable supporting lines are raised from the bottom plate 4, the middle moveable supporting line is lowered towards the bottom plate and both the fixed supporting lines remain fixed. The grid is then forced to assume a concave cylindrical shape that is defined by all the five supporting lines 14a-e. As the cylindrical shape is defined by as many as five supporting lines, a well defined shape is achieved and the grid is held well in place such that it is prevented from shaking of being displaced.

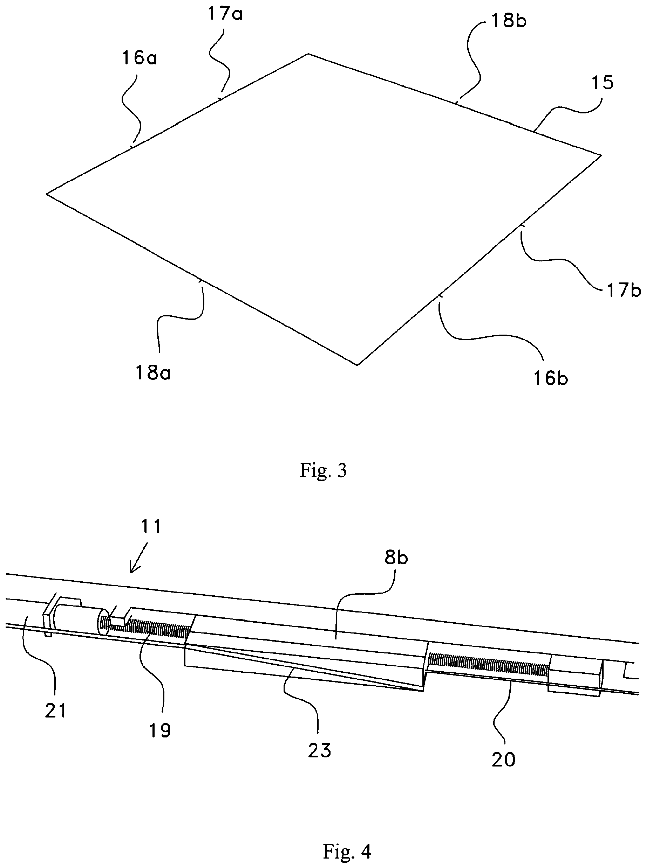

Fig. 3 shows the X-ray grid 15 with is six pegs 16a-b, 17a-b, 18a-b. Two pegs 16a-17a are arranged on one of a first pair of opposite sides and yet two pegs 16b-16b are arranged on the other of the first pair of opposite sides of the grid. These four pegs engage the linear actuators 12a-b, 13a-b along the first pair of opposite sides of the frame. Two pegs are 18a-b are arranged at the midpoint of two other opposite sides and these engage the linear actuators 8a-b on the second opposing sides of the frame.

Fig. 4 shows a linear actuator 8b being displaced by the control unit 11. The control unit comprises a motor 21 that turns a screw 19 that extends through an internally threaded bore through the linear actuator. The figure also illustrates the groove that extends diagonally across the linear actuator, which engages a peg on the grid. A wire 20 is attached to the linear actuator and extends from it in both directions. The wire is attached to all the linear actuators and extends all the way around the inner side of the frame.

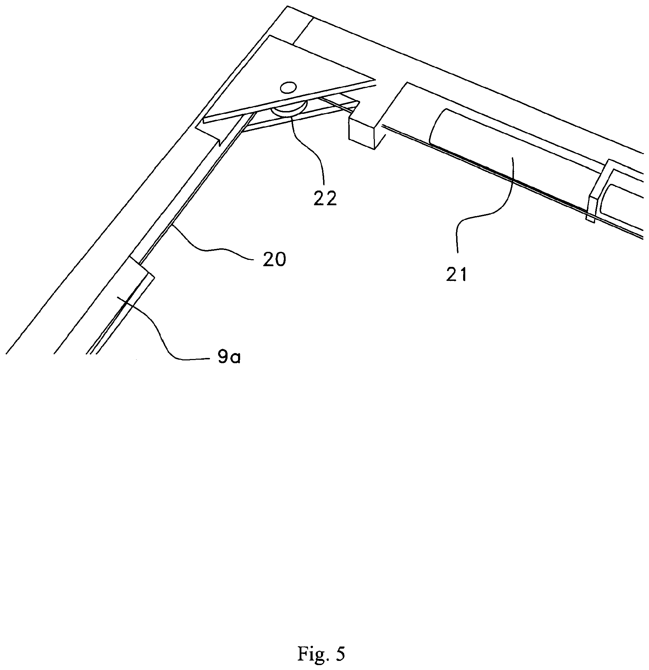

Fig. 5 shows part of the frame of the X-ray grid holder with a corner wheel 22 around which the wire 20 is arranged. Corresponding, easily rotatable corner wheel, are arranged in all the four corners such that the wire easily displaces all the linear actuators in a synchronous fashion.

## Claims

1. A holder for cylindrical bending of an X-ray grid (15), where the X-ray grid holder supports the X-ray grid with supporting elements (5a-b, 7a-b, 8a-b, 9a-b, 10a-b) arranged along five supporting lines (14a-e) being parallel to and separated from each other, where the supporting elements are constituted by moveable supporting elements (8a-b, 9a-b, 10a-b) and fixed supporting elements (5a-b, 7a-b), **characterised in that** the moveable supporting elements (8a-b) positioned along a mid supporting line (14c) are arranged to raise when the moveable supporting elements (9a-b, 10a-b) positioned along outermost supporting lines (14a-b) are lowered, while the fixed supporting elements (5a-b, 7a-b) positioned along the remaining two supporting lines stay fixed.

2. A grid holder according to claim 1, **characterised in that** said moveable supporting elements (9a-b, 10a-b) are moveably arranged along the sides of the grid holder and are provided with devices (23) for achieving raising or lowering of the grid when the moveable supporting elements are moved along the sides of the grid holder.

3. A grid holder according to claim 2, **characterised in that** said devices (23) for achieving raising or lowering of the grid are constituted by slanted grooves (23) in the moveable supporting elements, where the grooves are arranged to cooperate with pegs (16a-b, 17a-b, 18a-b) on the grid.

4. A grid holder according to any one of the previous claims, **characterised in that** said moveable supporting elements (9a-b, 10a-b) are mechanically connected to each other, such that all the moveable supporting elements move simultaneously.

5. A grid holder according to any one of the previous claims, **characterised in that** said fixed supporting elements (5a-b, 7a-b) are constituted by rails at the bottom and at the lid of the grid holder facing each other.

## Patentansprüche

1. Eine Halterung zum zylindrischen Biegen eines Röntgengitters (15), wobei die zylindrische Röntgengitterhalterung das Röntgengitter mit parallelen, voreinander getrennten Stützelementen (5a-b, 7a-b, 8a-b, 9a-b, 10a-b) unterstützt, die entlang von fünf Stützlinien (14a-e) angeordnet sind, wobei die Stützelemente als bewegliche Stutzelemente (8a-b, 9a-b, 10a-b) und feste Stützelemente (5a-b, 7a-b) ausgeführt sind, **dadurch charakterisiert,** dass die beweglichen Stützelemente (8a-b) so entlang einer mittleren Stützlinie (14c) angeordnet sind, dass sie angehoben werden wenn die beweglichen Stützelemente (9a-b, 10a-b), die entlang den äußeren Stützlinien (14a-b) angeordnet sind, abgesenkt werden, wobei die festen Stützelemente (5a-b, 7a-b), die entlang den zwei verbleibenden Stützlinien angeordnet sind, ortsfest bleiben.

2. Eine Gitterhalterung gemäß Anspruch 1, **dadurch charakterisiert,** dass besagte bewegliche Stützelemente (9a-b, 10a-b) beweglich entlang den Seiten der Gitterhalterung angeordnet und mit Verrichtungen (23) ausgestattet sind, die ein Anheben oder Absenken des Gitters ermöglichen, wenn die beweglichen Stützelemente entlang den Seiten der Gitterhalterung bewegt werden.

3. Eine Gitterhalterung gemäß Anspruch 2, **dadurch charakterisiert,** dass besagte Verrichtungen (23), die das Anheben oder Absenken des Gitters ermöglichen als schräge Nuten (23) in den beweglichen Stützelementen ausgeführt sind, wobei die Nuten so angeordnet sind, dass Stifte (16a-b, 17a-b, 18a-b) am Gitter in sie eingreifen.

4. Eine Gitterhalterung gemäß einem der vorstehenden Ansprüche, **dadurch charakterisiert,** dass besagte bewegliche Stützelemente (9a-b, 10a-b) so mechanisch untereinander verbunden sind, dass sich alle Stützelemente gleichzeitig bewegen.

5. Eine Gitterhalterung gemäß einem der vorstehenden Ansprüche, **dadurch charakterisiert,** dass besagte feste Stützelemente (5a-b, 7a-b) als Schienen auf dem Boden und an der gegenüberliegenden Klappe der Gitterhalterung ausgeführt sind.

## Revendications

1. Support permettant le pliage cylindrique d'une grille radiographique (15), où le support de la grille radiographique maintient la grille radiographique à l'aide d'éléments de support (5a-b, 7a-b, 8a-b, 9a-b, 10a-b) agencés le long de cinq axes de support (14a-e) parallèles et séparés les uns des autres, les éléments de support étant constitués d'éléments de support mobiles (8a-b, 9a-b, 10a-b) et d'éléments de support fixes (5a-b, 7a-b), **caractérisé en ce que** les éléments de support mobiles (8a-b) positionnés le long d'un axe de support médian (14c) sont agencés de façon à s'élever lorsque les éléments de support mobiles (9a-b, 10a-b) positionnés le long des axes de support les plus extérieures (14a-b) sont abaissés, alors que les éléments de support fixes (5a-b, 7a-b) positionnés le long des deux axes de supports restants, restent fixes.

2. Support de grille selon la revendication 1, **caractérisé en ce que** lesdits éléments de support mobiles (9a-b, 10a-b) sont agencés de façon à pouvoir se déplacer le long des côtés du support de grille, et sont munis de dispositifs (23) permettant de faire monter ou descendre la grille lorsque les éléments de support mobiles sont déplacés le long des côtés du support de grille.

3. Support de grille selon la revendication 2, **caractérisé en ce que** lesdits dispositifs (23) permettant la montée ou la descente de la grille sont constitués de rainures obliques (23) dans les éléments de support mobiles, les rainures étant agencées de façon à coopérer avec des chevilles (16a-b, 17a-b, 18a-b) sur la grille.

4. Support de grille selon l'une quelconque des revendications précédentes, **caractérisé en ce que** lesdits éléments de support mobiles (9a-b, 10a-b) sont mécaniquement reliés les uns aux autres, de façon à ce que tous les éléments de support mobiles se déplacent simultanément.

5. Support de grille selon l'une quelconque des revendications précédentes, **caractérisé en ce que** lesdits éléments de support fixes (5a-b, 7a-b) sont constitués de rails en bas et au niveau du couvercle du support de grille,1es uns en face des autres.
